Europäisches Patentamt

European Patent Office

Office européen des brevets

(19)

(11) Veröffentlichungsnummer: **0 038 868**
**B1**

(12) **EUROPÄISCHE PATENTSCHRIFT**

(45) Veröffentlichungstag der Patentschrift:
28.09.83

(51) Int. Cl.³: **A 61 K 7/16**

(21) Anmeldenummer: 80102305.2

(22) Anmeldetag: **29.04.80**

(54) Zahnpasta.

(43) Veröffentlichungstag der Anmeldung:
04.11.81 Patentblatt 81/44

(45) Bekanntmachung des Hinweises auf die Patenterteilung:
28.09.83 Patentblatt 83/39

(84) Benannte Vertragsstaaten:
AT BE CH DE FR GB IT LI LU NL SE

(56) Entgegenhaltungen:
DE-A-2 409 756
FR-A-1 360 156
FR-A-2 255 051
FR-M-3 652
GB-A-845 611
US-A-3 044 939
US-A-3 535 421
US-A-3 761 583
US-A-3 943 267
US-A-4 146 608
AADR Abstracts 1975, No. 117

Die Akte enthält technische Angaben, die nach dem
Eingang der Anmeldung eingereicht wurden und die
nicht in dieser Patentschrift enthalten sind.

(73) Patentinhaber: Blendax-Werke R. Schneider GmbH &
Co., Rheinallee 88, D-6500 Mainz (DE)

(72) Erfinder: Becker, Dieter, Messeler-Park-Strasse 21,
D-6100 Darmstadt-Wixhausen (DE)
Erfinder: Frosch, Franz, Dr., Eddersbacher Berg 6,
D-6201 Taunusstein (DE)
Erfinder: Harth, Helmuth, Dr., Am Gonsenheimer
Spiess 63, D-6500 Mainz (DE)
Erfinder: Raaf, Helmut, Dr., An der Schmalmach 1 B,
D-6208 Bad Schwalbach (DE)
Erfinder: Wagner, Helmar, R., Jägertorstrasse 108,
D-6100 Darmstadt-Arheilgen (DE)

**0 038 868**

## Zahnpasta

Die vorliegende Erfindung betrifft eine Zahnpasta, die eine ausgezeichnete zahnbelagsverhindernde Wirkung aufweist und damit einen wichtigen Beitrag zur Gesunderhaltung der Zähne und des Zahnfleisches leistet.

Daß Zahnbelag eine wesentliche Rolle sowohl bei der Entwicklung der Karies als auch der Parodontopathien spielt, ist heute in der zahnmedizinischen Wissenschaft unbestritten. Ein wesentlicher Teil der zahnmedizinischen Forschung ist deshalb darauf gerichtet, Substanzen und Mittel aufzufinden, die die Bildung von Zahnbelag auf den Zähnen verhindern vermögen. Dabei sind auch bereits wesentliche Fortschritte erzielt worden; eine der in dieser Hinsicht am besten untersuchten Verbindungen ist das unter dem Trivialnamen »Chlorhexidin« bekannte 1,6-Di-4'-(chlorphenyldiguanido)hexan, das nicht nur gegen die für die Bildung von Zahnbelag verantwortlichen Bakterien hervorragend wirkt, sondern auch auf den Zahnschmelz aufzieht und deshalb eine langdauernde Wirkung gewährleistet.

Leider weist das Chlorhexidin trotz seiner unbestreitbaren Erfolge insbesondere bei der Verhinderung bzw. Bekämpfung von Parodontopathien gewisse, an sich harmlose Nebenwirkungen auf, die bisher einer Daueranwendung in Zahn- und Mundpflegemitteln offenbar entgegenstanden:

Einerseits tritt beim längeren Gebrauch von Chlorhexidin enthaltenden Präparaten eine an sich harmlose Verfärbung der Zähne und der Schleimhäute auf, die zwar mechanisch beseitigt werden kann, trotzdem jedoch kosmetisch störend wirkt; darüber hinaus kann es auch zu Geschmacksirritationen kommen.

Es wurde deshalb versucht, Substanzen aufzufinden, die diese Nebenwirkungen nicht aufweisen, jedoch gleichwohl die Bildung von Zahnbelag wirksam verhindern können.

In diesem Zusammenhang ist auch bereits die Wirksamkeit von Kupferionen festgestellt worden, vgl. AADR Abstracts 1979, No. 117 (Journal of Dental Research, 1979, Special Issue A, S. 74).

Obwohl Kupferionen liefernde Verbindungen in Lösungen entsprechend der zitierten Vorveröffentlichung gute Resultate bei der Verhinderung von Zahnbelag ergeben haben, ist es überraschenderweise bislang nicht möglich gewesen, diese Resultate auch mit entsprechenden Zahnpasten zu erhalten.

Die vorliegende Erfindung geht daher von der Aufgabenstellung aus, eine Pasta mit einem Gehalt an gegen Zahnbelag wirksamen Kupferverbindungen zu entwickeln, die auch als Zahnpasta gegen die Bildung von Zahnbelag wirksam ist.

Die Lösung dieser Aufgabe besteht erfindungsgemäß darin, daß eine Zahnpasta auf Basis üblicher Grund- und Zusatzstoffe verwendet wird, die mindestens eine Kupferverbindung und mindestens ein Poliermittel enthält und dadurch gekennzeichnet ist, daß das Poliermittel zumindest zu mehr als der Hälfte aus einem feinteiligen Kunststoff besteht.

Überraschenderweise zeigt sich nämlich, daß gerade bei der Verwendung dieser Poliermittel eine Zahnpasta mit einem Gehalt an einer an sich bekannten Kupferverbindung eine hervorragende Wirksamkeit gegen Zahnbelag aufweist.

Bei den eingesetzten feinteiligen, pulverförmigen Kunststoff-Poliermitteln handelt es sich um die an sich für diesen Zweck bereits seit längerem bekannten Produkte.

Sie weisen vorzugsweise einen durchschnittlichen Teilchendurchmesser von etwa 0,5 bis etwa 40 Mikron, vorzugsweise etwa 2 bis etwa 20 Mikron, auf und sind in den Zahnpasten in einer Menge von etwa 20 bis etwa 60 Gew.-% enthalten.

Als bevorzugtes Poliermittel gilt Polymethylmethacrylat, vorzugsweise mit einem mittleren Teilchendurchmesser zwischen etwa 0,2 und etwa 5 Mikron.

Es können jedoch auch andere, teilweise an sich zum Einsatz in Zahnpasten bereits vorgeschlagene Kunststoffe wie Polyvinylchlorid, Polystyrol, Polycarbonat, Copolymerisate aus Methylmethacrylat und anderen Comonomeren, die die Härte und Abrasivität des Polymethylmethacrylats nicht negativ beeinflussen, wobei der Anteil an Methylmethacrylat im Copolymerisat vorzugsweise bei mindestens 80% liegt, Polyamide, beispielsweise pulverförmiges Nylon, Harnstoff-Formaldehyd-Harze, Melamin-Formaldehyd-Harze, Phenol-Formaldehyd-Harze, pulverförmige, ausgehärtete Epoxyharze, verschiedene Polyester etc. Einsatz finden.

Geeignete feinverteilte Kunststoff-Poliermittel sind beispielsweise in den US-PS 2 130 034, 3 070 510, 3 151 027, 3 251 800 und 3 357 950 sowie der DE-AS 1 617 306 beschrieben.

Vorzugsweise gelangt das feinteilige Kunststoffpulver in der erfindungsgemäßen Zahnpasta als alleiniges Poliermittel zum Einsatz.

Es ist jedoch auch möglich, andere Poliermittel in untergeordneten Mengen (d. h., in jedem Fall weniger als die Hälfte des gesamten Poliermittels ) zuzusetzen, sofern diese nicht inaktivierend auf die vorhandenen Kupferionen einwirken.

Derartige Poliermittel sind beispielsweise Alkalialuminiumsilikate sowie insbesondere die verschiedenen Siliciumdioxide, beispielsweise durch Fällung erhaltene Kieselsäuren, wie sie in den DE-OS 2 206 285, 2 446 038 und 2 610 207 oder den GB-PS 1 433 743 und 1 447 663 und der US-PS 4 122 160 beschrieben und beispielsweise unter den Bezeichnungen »Neosyl«® oder »Sident«® im Handel sind.

Ebenfalls geeignet für den erfindungsgemäßen Zweck sind die in der US-PS 3 538 230 beschriebenen Siliciumdioxid-Xerogele mit Oberflächen zwischen 150 und etwa 800 m²/g, wie sie beispielsweise von der Firma Grace & Co. unter der Bezeichnung »Syloid«® vertrieben werden.

Auch teilweise dehydratisierte Siliciumdioxid-Hydrogele, wie sie aus den DE-OS 2 704 504 und 2 920 906 bekannt sind, können in den erfindungsgemäßen Zahnpasten als untergeordneter Poliermittelbestandteil eingesetzt werden.

Die Teilchengrößen dieser gegebenenfalls zusätzlich anwesenden Poliermittel liegen in der Regel ebenfalls in dem für diesen Zweck üblichen und optimalen Bereich, das heißt, zwischen etwa 1 und 20 Mikron, vorzugsweise zwischen etwa 3 und etwa 14 Mikron.

Die Menge an Kupferverbindungen, die in den erfindungsgemäßen Zahnpasten zum Einsatz gelangt, sollte so bemessen sein, daß etwa 0,001 bis etwa 5 Gew.-% Cu, berechnet auf die gesamte Zahnpasta, anwesend sind.

Ein bevorzugter Mengenbereich liegt bei 0,05 bis 1,5%, insbesondere bis etwa 0,5% Cu.

Als Kupferionen liefernde Kupferverbindungen sind prinzipiell alle toxikologisch unbedenklichen, schleimhautverträglichen, auch nur einigermaßen wasserlöslichen Kupferverbindungen geeignet.

Von den anorganischen Kupfersalzen seien beispielhaft genannt: Kupferchlorid, $CuCl_2$, und dessen Dihydrat; Kupferfluorid, $CuF_2$, und dessen Dihydrat; Kupferfluosilikat, $CuSiF_6$, und dessen Hexahydrat; Kupfersulfat, $CuSO_4$, und dessen Pentahydrat; Kupfernitrat bzw. dessen Tri- und Hexahydrat sowie auch ausgefallenere Kupfersalze wie Kupferbromid, $CuBr_2$; Kupfermetaborat, $Cu(BO_2)_2$; Kupferbromat $Cu(BO_3)_2 \cdot 6 H_2O$; Kupferchlorat, $Cu(ClO_3)_2 \cdot 6 H_2O$; Kupferjodat, $Cu(IO_3)_2$, und Kupferfluorphosphat, $CuPO_3F$.

Bevorzugte Kupfersalze organischer Säuren sind Kupferacetat, Kupferformiat, Kupferbenzoat, Kupfercitrat, Kupfertartrat, Kupferlactat, Kupfermalat, Kupfermandelat, Kupfersorbat, Kupferpantothenat, Kupfergluconat, Kupferphytat, Kupferglycerophosphat, Kupfercinnamat, Kupferbutyrat, Kupferpropionat, Kupferlaurat, Kupferoxalat und Kupfersalicylat.

Wie bereits erwähnt, enthalten die erfindungsgemäßen Zahnpasten neben dem obligatorischen feinteiligen Kunststoff-Poliermittel und einer oder mehreren Kupferverbindungen übliche Zusatz- und Grundstoffe.

Hier sind insbesondere die bekannten Feuchthaltemittel zu nennen, insbesondere Glycerin und andere Polyalkohole wie Propylenglycol, 1,3-Butandiol und Polyethylenglycole mit niederem Molgewicht sowie Zuckeralkohole, insbesondere Sorbit und gegebenenfalls auch Mannit und Xylit.

Darüber hinaus enthalten Zahnpasten üblicherweise Verdickungs- und Bindemittel. Im vorliegenden Fall eignen sich am besten Cellulosederivate, insbesondere Hydroxyalkylcellulose wie Hydroxyethylcellulose und Pflanzengummen, wie Xanthan Gum, Irish Moos und gegebenenfalls auch gegenüber Kupferionen inerte anorganische Verdickungsmittel, deren Anteil üblicherweise zwischen etwa 0,25 und etwa 3,5 Gew.-% der Zahnpasta beträgt.

Die erfindungsgemäßen Zahnpasten können auch oberflächenaktive Substanzen enthalten. Diese werden insbesondere zur Erzeugung einer vom Verbraucher geschätzten Schaumkraft eingesetzt. Geeignete oberflächenaktive Substanzen sind besonders wasserlösliche Salze von höheren Alkylsulfaten oder Alkylethersulfaten, beispielsweise Natriumlaurylsulfat, aliphatische Acylamide gesättigter Monoaminocarbonsäuren, vorzugsweise Natrium-N-lauroylsarcosinat, Taurin-Fettsäureamide, beispielsweise Natrium-N-alkyl-N-myristoyltaurid, Salze von sulfonierten Monoglyceriden höherer Fettsäuren, beispielsweise Natriummonoglyceridsulfonat, Fettsäureester der Isäthionsäure und deren Salze, nichtionische Tenside wie Alkylenoxidkondensate mit Fettalkoholen und ein- oder mehrwertigen Aminen, Zuckerester, beispielsweise Saccharosemonolaurat, Sorbitolpolyoxyäthylenstearat, langkettige Aminoxide, beispielsweise Dimethyllaurylaminoxid, ampholytische Tenside, beispielsweise Betaine oder langkettige Alkylaminocarbonsäuren, und kationaktive Tenside, beispielsweise quartäre Ammoniumverbindungen wie Cetyltrimethylammoniumbromid.

Der Anteil an oberflächenaktiven Verbindungen in der erfindungsgemäßen Zahnpasta liegt bei 0 bis etwa 5 Gew.-% der Gesamtzusammensetzung.

Zahnpasten enthalten üblicherweise Aroma- und Geschmackstoffe, Konservierungsmittel etc., derartige Mittel sind an sich bekannt und in zahlreichen Druckschriften beschrieben.

Es ist eine bevorzugte Ausführungsform der Erfindung, Fluorverbindungen in der erfindungsgemäßen Zahnpasta mitzuverwenden, vorzugsweise in solchen Mengen, daß die Konzentration an reinem Fluor im Mittel 0,01 bis 1 Gew.-%, vorzugsweise 0,1 bis 0,5 Gew.-% der Zahnpasta beträgt.

Geeignete Fluorverbindungen sind insbesondere die verschiedenen Salze der Monofluorphosphorsäure, insbesondere Natrium-, Kalium-, Lithium-, Calcium- und Aluminiummono- und -difluorphosphat, sowie die verschiedenen, Fluor in ionisch gebundener Form enthaltenden Fluoride, insbesondere Alkalifluoride wie Natrium-, Lithium-, Kalium- und Ammoniumfluorid, Zinnfluorid, Manganfluorid, Zirkoniumfluorid und Aluminiumfluorid sowie Gemische oder Anlagerungsprodukte dieser Fluoride untereinander oder mit anderen Fluorverbindungen, beispielsweise Kalium- oder Natriummanganfluorid.

Andere im Rahmen der vorliegenden Erfindung einsetzbare Fluoride sind beispielsweise Zinkfluorid, Germaniumfluorid, Palladiumfluorid, Titanfluorid, Alkalifluorzirkonate, beispielsweiwe Natrium- oder Kaliumfluorzirkonat, Zinnfluorzirkonat, Fluorborate oder Fluorsulfate, beispielsweise Natrium- oder Kaliumfluorosulfat.

Fluor- und Kupferionen können in der erfindungsgemäßen Zahnpasta auch durch eine Verbindung zur Verfügung gestellt werden, solche Verbindungen sind beispielsweise Kupferfluorid, Kupfermonofluorphosphat und Kupferfluorsilikat.

Auch organische Fluorverbindungen können mit Erfolg eingesetzt werden, insbesondere die bekannten Additionsprodukte aus langkettigen Aminen oder Aminosäuren und Fluorwasserstoff, Monoäthanolaminohydrofluorid oder Methyltriäthylammoniumfluorid.

Die erfindungsgemäßen Zahnpasten können weitere, zur Verwendung in solchen Mitteln an sich bekannten Stoffe enthalten, beispielsweise Enzyme wie Proteasen und Carbohydrasen, z. B. Amylase, Dextranase, Lävanase oder $\alpha$-1,3-Glucan-3-glucanohydrolase, Zahnstein entfernende Substanzen wie die für diesen Zweck vorgeschlagenen Phosphonsäuren, beispielsweise Hydroxyäthan-1,1-diphosphonsäure, oder die als Zahnbelag verhindernde Substanzen bekannten Bisbiguanide bzw. deren vorzugsweise wasserlöslichen Salze.

Eine ausführliche Übersicht über die Herstellung von Zahnpflegemitteln und die dabei zum Einsatz gelangenden Stoffe findet sich in dem Handbuch von M. S. Balsam und E. Sagarin, »Cosmetics — Science and Technology«, 2nd Ed., Vol. 1, S. 423 bis 531 (1972).

Im folgenden werden einige Beispiele für erfindungsgemäß zusammengesetzte Zahnpasten gegeben.

### Beispiel 1

| | |
|---|---|
| Xanthan Gum | 0,90 (Gew.-%) |
| Glycerin | 15,00 |
| Sorbit | 12,00 |
| $CuSO_4 \cdot 5 H_2O$ | 0,40 |
| Natriummonofluorphosphat | 0,76 |
| Natriumlaurylsulfat | 1,40 |
| Polymethylmethacrylat-Pulver (mittlerer Teilchendurchmesser ~4 μm) | 42,00 |
| Pyrogenes Siliciumdioxid | 2,20 |
| Aromagemisch | 1,00 |
| Saccharin-Natrium | 0,25 |
| p-Hydroxybenzoesäuremethylester | 0,20 |
| Entsalztes Wasser | 23,89 |

### Beispiel 2

| | |
|---|---|
| Irish Moos | 0,50 (Gew.-%) |
| Xanthan Gum | 0,50 |
| Glycerin | 7,50 |
| Sorbit | 22,00 |
| Kupferformiat $\cdot$ 4 $H_2O$ | 0,30 |
| Kupferfluorid ($CuF_2$) | 0,25 |
| Natriumlauroylsarcosinat | 1,40 |
| Gehärtetes Melamin-Formaldehyd-Kondensat (mittlerer Teilchendurchmesser ~1 – 10 μm) | 28,50 |
| Titandioxid | 0,50 |
| Saccharin-Natrium | 0,10 |
| Aromagemisch | 1,00 |
| p-Hydroxybenzoesäuremethylester | 0,10 |
| p-Hydroxybenzoesäure-n-propylester | 0,05 |
| Entsalztes Wasser | 37,30 |

### Beispiel 3

| | |
|---|---|
| Hydroxyäthylcellulose | 1,10 (Gew.-%) |
| Glycerin | 10,00 |
| Sorbit | 20,00 |
| Kupferfluorsilikat ($CuSiF_6 \cdot 6 H_2O$) | 0,95 |
| Natriumlaurylsulfat | 1,50 |
| Feinverteiltes Polymethylmethacrylat-Pulver (mittlerer Teilchendurchmesser ~0,5 – 5 μm) | 25,00 |

| | |
|---|---|
| Siliciumdioxid-Aerogel (Syloid® 244, Oberfläche etwa 260 m$^2$/g) | 1,80 |
| Polyvinylchlorid-Pulver (mittlerer Teilchendurchmesser ~1 – 10 µm) | 7,30 |
| Aromagemisch | 1,00 |
| Saccharin-Natrium | 0,15 |
| p-Hydroxybenzoesäuremethylester | 0,20 |
| Entsalztes Wasser | 31,00 |

## Beispiel 4

| | |
|---|---|
| Methylcellulose | 1,20 (Gew.-%) |
| 1,3-Butandiol | 6,00 |
| Glycerin | 13,00 |
| Sorbit | 10,00 |
| Natriumlaurylsulfat (10%ige Aufschlämmung in Glycerin) | 3,50 |
| Kupferfluorid (CuF$_2$ · 2 H$_2$O) | 0,30 |
| Kupferpantothenat | 0,80 |
| Feinverteiltes Harnstoff-Formaldehyd-Kondensat | 35,00 |
| gehärtet (mittlerer Teilchendurchmesser ~2 – 8 µm) | 2,00 |
| Aromagemisch | 1,20 |
| Saccharin-Natrium | 0,20 |
| p-Hydroxybenzoesäureäthylester | 0,13 |
| Benzoesäure | 0,10 |
| Weinsäure | 0,55 |
| Entsalztes Wasser | 26,02 |

## Beispiel 5

| | |
|---|---|
| Glycerin | 19,00 (Gew.-%) |
| Sorbit (70%ig) | 7,00 |
| Polyäthylenglykol 300 | 3,00 |
| Kupferlactatdihydrat | 1,20 |
| Zinnfluorid (SnF$_2$) | 0,40 |
| Hydroxyäthan-1,1-diphosphonsäure, Trinatriumsalz | 1,25 |
| Bromchlorophen | 0,05 |
| Benzoesäure | 0,15 |
| Dehydracetsäure | 0,10 |
| p-Hydroxybenzoesäure-n-propylester | 0,05 |
| Polymethylmethacrylatpulver (mittlerer Teilchendurchmesser ~3 – 8 µm) | 20,00 |
| Siliciumdioxid-Xerogel (vom Typ Syloid® 70, Oberfläche etwa 290 m$^2$/g) | 8,50 |
| Pyrogenes Siliciumdioxid (vom Typ Aerosil®) | 1,20 |
| Natriumlaurylethersulfat (25%ig in Ethanol) | 10,00 |
| Etnsalztes Wasser | 27,30 |
| Xanthan Gum | 0,80 |

## Patentansprüche

1. Zahnpasta auf wäßriger Basis mit üblichen Grund- und Zusatzstoffen, enthaltend mindestens ein Poliermittel und 0,001 bis 5 Gew.-%, bezogen auf die Gesamtzusammensetzung (berechnet als Cu), mindestens einer Kupferionen liefernden Verbindung, dadurch gekennzeichnet, daß das Poliermittel zu mehr als der Hälfte aus einem feinverteilten Kunststoffmaterial besteht.

2. Zahnpasta nach Anspruch 1, dadurch gekennzeichnet, daß das Poliermittel zumindest teilweise aus feinverteiltem Polymethylmethacrylat besteht.

3. Zahnpasta nach Anspruch 1, dadurch gekennzeichnet, daß das Poliermittel zumindest teilweise aus einem feinverteilten Aminoplast-Harz besteht.

4. Zahnpasta nach einem der vorhergehenden Ansprüche, dadurch gekennzeichnet, daß sie als Kupferionen liefernde Verbindung ein wasserlösliches, anorganisches Kupfersalz enthält.

5. Zahnpasta nach einem der Ansprüche 1 bis 3, dadurch gekennzeichnet, daß sie als Kupferionen liefernde Verbindung ein Kupfersalz einer organischen Säure enthält.

6. Zahnpasta nach Anspruch 5, dadurch gekennzeichnet, daß sie als Kupfersalz einer organischen Säure mindestens eine der Verbindungen Kupfercitrat, Kupfertartrat, Kupferpantothenat, Kupferlactat, Kupfermalat, Kupfermandelat, Kupfersorbat, Kupferbenzoat, Kupfersalicylat, Kupfergluconat, Kupferphytat, Kupferglycerophosphat und/oder Kupfercinnamat enthält.

7. Zahnpasta nach einem oder mehreren der vorhergehenden Ansprüche, dadurch gekennzeichnet, daß sie die Kupferverbindung in einer Menge von 0,05 bis 0,5 Gew.-% (berechnet als Cu), bezogen auf die Gesamtzusammensetzung, enthält.

## Claims

1. Aqueous toothpaste composition containing usual compounds, at least one polishing agent and 0,001 to 5% by weight of the total composition (calculated on Cu) of at least one copper ions releasing compound, characterized in that more than fifty percent of the polishing agent are composed of a finely divided plastic material.

2. Toothpaste according to claim 1, characterized in that the polishing agent is composed at least partially of finely divided polymethyl methacrylate.

3. Toothpaste according to claim 1, characterized in that the polishing agent is composed at elast partially of finely divided aminoplast resin.

4. Toothpaste according to one of the preceding claims, containing as copper ions releasing compound a watersoluble inorganic copper salt.

5. Toothpaste according to one of the claims 1 to 3, containing as copper ions releasing compound a copper salt of an organic acid.

6. Toothpaste according to claim 5, containing as copper salt of an organic acid at least one of the compounds copper citrate, copper tartrate, copper pantothenate, copper lactate, copper malate, copper mandelate, copper sorbate, copper benzoate, copper salicylate, copper gluconate, copper phytate, copper glycerophosphate and/or copper cinnamate.

7. Toothpaste according to one or more of the preceding claims, characterized in that it contains 0,05 to 0,5% by weight of the total composition (calculated on Cu) of the copper compound.

## Revendications

1. Composition de pâte dentifrice aqueuse contenant des matières habituelles, au moins un agent de polissage et 0,001 à 5% de poids (calculé comme Cu) d'au moins un composé livrant des ions de cuivre, caractérisée par le fait que plus que 50% de l'agent de polissage se composent d'une matière plastique distribuée fine.

2. Pâte dentifrice selon revendication 1, caractérisée par le fait que l'agent de polissage se compose au moins partiellement de polyméthyl méthacrylate distribué fine.

3. Pâte dentifrice selon revendication 1, caractérisée par le fait que l'agent de polissage se compose au moins partiellement d'un résin aminoplaste.

4. Pâte dentifrice selon une des revendications précédentes, caractérisée par le fait qu'elle contient comme un composé livrant des ions de cuivre du sel de cuivre inorganique soluble dans l'eau.

5. Pâte dentifrice selon une des revendications 1 à 3, caractérisée par le fait qu'elle contient comme un composé livrant des ions de cuivre du sel de cuivre d'une acide organique.

6. Pâte dentifrice selon revendication 5, caractérisée par le fait qu'elle contient comme sel de cuivre d'une acide organique au moins un des composés citrate de cuivre, tartrate de cuivre, pantothénate de ciuvre, lactate de cuivre, malate de cuivre, mandélate de cuivre, sorbate de cuivre, benzoate de cuivre, salicylate de cuivre, gluconate de cuivre, phytate de cuivre, glycerophosphate de cuivre et/ou cinnamate de cuivre.

7. Pâte dentifrice selon une ou plusieurs des revendications précédentes, caractérisée par le fait qu'elle contient 0,05 à 0,5% de poids de la composition totale (calculé comme Cu) du composé de cuivre.